# EUROPEAN PATENT SPECIFICATION

(11) **EP 0 313 833 B1**
(45) Date of publication and mention of the grant of the patent: **07.01.1993**
(21) Application number: 88115770.5
(22) Date of filing: 24.09.1988
(51) Int. Cl.: G01N 33/53, G01N 33/543

(54) **Disposable pre-packaged device for conducting immunoassay procedures**
Vorverpackte Einwegvorrichtung für Immuntestprozeduren
Dispositif jetable préemballé pour exécuter des procédés d'essai immunologiques

(30) Priority: 29.10.1987 US 107240
(43) Date of publication of application: 03.05.1989
(73) Proprietor: Hygeia Sciences, Inc., Newton Massachusetts 02160-1432 (US)
(72) Inventor: Lennon, Donald J., Hopedale, MA 01747 (US); Murphy, Timothy C. Jr., Westwood, MA 02090 (US)
(74) Representative: König, Werner, Dipl.-Ing.

(56) References cited:
- EP-A- 0 253 579
- WO-A-85/05451
- US-A- 3 888 629
- US-A- 4 180 383
- US-A- 4 246 339

## Description

### BACKGROUND OF THE INVENTION:

### Field of the Invention:

The present invention relates to devices for conducting immunoassay procedures. In particular, the invention relates to disposable, pre-packaged devices which are particularly suitable for conduct of diagnostic procedures based on immunological reactions at remote sites such as physician's offices and homes of users.

### Description of the Prior Art:

Since the important discovery of Millstein and Kohler reported in Nature 256: 495-497, 1975, the development of highly sensitive and specific immunoassay procedures has proceeded at a rapid pace. In fields such as clinical medicine, forensic sciences, environmental quality testing, food quality assurance, drug testing and other related areas, it has become possible to determine the presence and/or amount of trace substances in test samples even when such substances are present in very low concentrations in the order of parts per million, or even less. The development of non-radioactive labels or markers, such as enzyme color formers, has facilitated the use of immunoassay diagnostic procedures outside of laboratory settings and in remote sites such as physician's offices and even the homes of the users. In the physician's office, immunological procedures are useful to provide rapid, simple assays which may be performed while the patient is still in the office so that the diagnosis can be accomplished without delay and treatment instituted during a single visit. Without such simple assays, it has often been necessary for the physician to collect a sample from the patient during a first visit and to have the sample analyzed by a clinical laboratory with the results reported back to the physician by the laboratory at a later time. In the meanwhile, the patient was sent home and was required to return for a second visit with the physician in order to receive appropriate treatment and/or medication. Manifestly, such delay was inefficient and inappropriate and in some cases could even be life threatening.

Home testing has become desirable to facilitate testing by the consumer in the privacy of his or her own home. The results of such testing might, for example, indicate the necessity or lack of necessity of a visit to the physician. Examples of useful test for the "at home" market include tests for pregnancy, ovulation, streptococcus infection and other infections which are detectable by analysis of urine, saliva or other appropriate test samples.

For remote site testing, assuming appropriate sensitivity and specificity can be achieved, there are at least three other requirements for practical assay procedures. The first of these desirable factors is speed in that the assay must be performed in an acceptably short period of time, the shorter the better. Stability is also a desirable feature in that the components of the assay should be stable for an extended period of time without refrigeration or special handling and the assay results or readouts should be sufficiently stable so that the interpretation may be confirmed even several days after the initial test is performed. Finally, from a commercial view point it is desirable that the test be as simple as possible requiring only minimal or no instrumentation and precluding mistakes and poor performance resulting in incorrect interpretations.

Immunoassay kits employing enzyme markers are presently commercially available today for determining pregnancy and ovulation in the physician's office and in the home of the user. The technical components generally required in such kits are (1) a solid phase bearing immobilized antibody, (2) an enzyme labelled antibody, (3) a rinse solution (in some cases this may be the users tap water), and (4) a substrate for the enzyme. A typical procedure is that the sample is mixed with the solid phase and incubated (with or without a subsequent rinse step) and then the sample is discarded, the solid phase is then contacted with the enzyme labelled antibody and incubated. Thereafter the solid phase is rinsed and contacted by the substrate. After a period of time (ca 5 minutes) the color of the solid phase is observed. One such assay is described in United States Letters Patent No. 4,632,901.

Enzyme labelled immunoassays are not without their own drawbacks resulting from the instability of some enzyme systems, the number of kit components and the complexity of the procedure.

One of the difficulties encountered in the development of test devices for remote site testing is the provision of a practical pre-packaged disposable device to facilitate efficient, relatively inexpensive test procedures. This, of course, requires a device which is inexpensive to construct, which has a shelf life appropriate to the commercial use of the device, which is protected against contamination during handling, and which may be simply and readily utilized when the appropriate time arises. The device illustrated in US patent No. 4,632,901 mentioned above addresses some of these problems and is available commercially; however, the device has a number of deficiencies including retarded flow of fluid into the absorbent. It is believed that such flow problems were perhaps alleviated to some degree by the provision of notched vertical venting grooves in the periphery of the absorbent plug. Such venting grooves facilitated venting of the device during use through small ports provided near the bottom of the container. Of course, liquid could escape through such vent ports creating a messy operation, sealing prior to use was difficult and the manufacture of the grooved absorbent plugs is a relatively expensive operation.

Other prior single test devices are illustrated in European Application EP-A-253 579, in PCT Application WO-85/05451 and in the five U.S. Letters Patent Nos. 3,888,629, 4,180,383, 4,246,339, 4,366,241 and 4,623,461.

Earlier European Application EP-A-253 579 published on January 20, 1988 has the priority date of Juli 14, 1986 and is therefore considered pursuant to article 54(3) and (4) EPC, as comprised in the state of the art. In this application a device is disclosed comprising a container for receiving liquid residues, a liquid absorbing zone in the container, a porous media means for capturing and displaying products to be measured, a said porous capture means being disposed adjacent the inlet of the container and in fluid communication with absorbent means, and a removable plug having a centrally disposed cylindrical member extending through the containers inlet and defining a throat positioned for directing flow of liquid through the inlet and onto the capture means, said cylindrical member having an annular surface at its free end disposed for contacting the porous capture media means and pushing the latter towards the absorbent means. But no spacer means do exist which could define a breathing space extending entirely around the absorbent means. Means for venting air only consist of at least one vent port, whereby these vent ports are formed of grooves in the container.

WO-85/05451 discloses a device comprising a container for receiving liquid residues, a body of absorbent material, and a porous capture media means, said absorbent material and capture means being placed in the container. Means for venting air, e.g. small ports, may be placed in the container near the bottom. This container has also no spacer means and also no lid on the device.

The U.S. Letters Patent No. 3,888,629 discloses a three-part device comprising a middle part, which contains a matrix pad of absorbent material for carrying out a desired reaction, a lower part which contains an absorbent material to abut the matrix pad and promote filtration through the pad after the reaction has taken place, and an upper part functioning as a reservoir chamber. The upper part may function as a lid and the lower part (container) may contain a central drain hole in the bottom. There is no spacer means in the lower part.

The U.S. Letters Patent No. 4,246,339 discloses a device comprising a telescoping top means with one or more wells with bottom openings covered with microporous membranes, and a bottom member defining a liquid reservoir containing absorbent means. The top means may contain holes for venting air. There is no spacer means in the bottom member.

The U.S. Letters Patent No. 4,180,383 discloses a device comprising means forming a supporting structure, means defining a chamber in said structure, an immobilized immunoadsorbent positioned within said chamber, a porous plug positioned within said chamber, means forming an inlet (lid) and an outlet for passage of fluid through said chamber. There is no spacer means in the chamber.

Finally the devices described in the U.S. Letters Patent Nos. 4,366,241 and 4,623,461 are of limited application and also have excessive complexity.

### Summary of the Invention

The present invention provides relief from many of the shortcomings of the prior art devices described above. In this regard, the invention provides a simplified, pre-packaged, disposable test device for conducting an immunoassay procedure which results in the production of separable liquid and solid phases, whereby the liquid sample flows freely through a porous capture media means where products of the procedure are captured and displayed.

The device comprises structural elements which facilitate assembly, pre-packaging and sealing of the device prior to intended use like cup means defining an elongated chamber for receiving the liquid phase from said procedure, said cup means comprising a cylindrical wall extending around said chamber, a closure wall at one end of the chamber and a liquid inlet communicating with the chamber, further a generally cylindrically shaped absorbent plug disposed entirely within the chamber for absorbing said liquid phase, said plug having a lower surface resting on said closure wall, a circumferential peripheral surface extending therearound and an upper flat surface disposed in alignment with said inlet and finally a porous capture media means for capturing and displaying a solid phase separated from said liquid phase, said porous capture media means being disposed between said inlet and said flat surface and being in fluid communication with said absorbent plug via said flat surface, whereby said absorbent plug promotes flow of liquid phase through said capture media means during conduct of an immunoassay procedure.

In accordance with the present invention a spacer means is provided in said chamber holding the peripheral surface of the absorbent plug away from the cylindrical wall to thereby present an annular breathing space communicating directly with the peripheral surface of the absorbent plug and extending essentially entirely around said peripheral surface to facilitate venting and thus flow of fluid into the absorbent during the conduct of the test prcedure.

In a most desirable commercial form of the invention, the device includes a lid element mounted on the cup means adjacent the inlet and having a centrally disposed, elongated cylindrical member extending through the inlet, said element having an opening extending through the cylindrical member for directing flow of liquid assay reaction medium through the inlet and onto the capture means and including at least one vent hole adjacent said inlet in communication with said space to facilitate venting of the absorbent plug via said peripheral surface and said space as the plug absorbs a liquid phase. The cylindrical member has an annular surface at its free end disposed for contacting the porous capture media means and pushing the latter towards the absorbent means and into said fluid communication.

To facilitate manufacture and assembly of the device, the cylindrical member includes a pusher segment for contacting said capture means and excerting a pushing force against the latter to hold the capture means and the plug in said fluid communication therewith.

Also, to facilitate assembly the lid element preferably includes an annular flange extending around said opening and the cup means includes a mating, corresponding annular flange extending around said inlet and said device including means for securing the flanges together in mated relationship with the opening aligned with the inlet.

To further facilitate pre-packaging of the device, each vent hole is disposed in said lid element flange and therewith in fluid communication with the breathing space of the cup device and facilitating venting of the latter to the atmosphere.

Such construction enables the use of a removable sealing means closing the opening and each vent hole to thereby prevent introduction of foreign material into the device prior to intended use.

In this regard, in a preferred embodiment of the invention the external portions of the opening and the vent holes are disposed in a common plane and said sealing means comprising a single planar element disposed in sealing relationship to said external portions.

In a most desirable commercial form of the invention the pusher segment is cylindrical and has an annular surface at its free end disposed for contacting the porous capture media means, said opening extending through the cylindrical pusher segment.

In one preferred form of the invention, the porous media means comprises a filter element having pores of a size to prevent passage of desired reaction products.

In another preferred form of the invention, the porous media means comprises a microporous membrane to which is bound an immunoreactive substance that is specifically reactive in the desired reaction.

In either case, the device preferably includes a porous separator element which is interposed between the porous means and the flat surface of the absorbent plug wherein said separator element can be constructed of a hydrophobic material to insure complete separation between the captured reaction product on the capture media means and the liquid phase which has been drawn into the absorbent means.

In a particularly preferred form of the invention, the plug is generally circular in cross-sectional configuration and said spacer means comprises a plurality of general rectangular shaped wing members extending radially inwardly from said cylindrical wall.

### BRIEF DESCRIPTION OF THE DRAWINGS:

Figure 1 is an isometric view of a disposable, pre-packaged device for conducting an immunoassay procedure, which device embodies the principles and concepts of the present invention;
Figure 2 is a cross-sectional view taken along view line 2-2 of Figure 1;
Figure 3 is a cross-sectional view of the device of the invention taken along view line 3-3 of Figure 2;
Figure 4 is an elevational, exploded view of the device illustrating the relationship between the various components of the assembled device; and
Figure 5 is a cross-sectional view of an alternative form of the lid element useful in connection with an alternative device embodying the invention.

### DETAILED DESCRIPTION OF THE INVENTION

The concepts and principles of the present invention are embodied in a disposable, pre-packaged device 10 which is useful for conducting an immunoassay procedure. The type of procedure utilized is not important for purposes of the present invention other than that the device may be used to facilitate any sort of procedure which results in the production of a collectible phase which incorporates a tag of some sort to indicate a positive or negative test results. Generally speaking, the device 10 of the present invention will be utilized in connection with procedures which employ a visually detectable colored or color forming tag such as an enzyme or metal sol particle tag. However, it is within the perceived usefulness of the invention that the device might well be employed in connection with procedures wherein a reactant is tagged with an instrument detectable tag such as a radioactive isotope, a fluorescent material or a chemiluminescent material.

The device 10, in its preferred form, includes a cup member 12 encompassing a chamber 14 for receiving liquid residues from an immunoassay procedure. Absorbent means in the form of an elongated absorbent plug 16, which is generally circular in cross-sectional configuration, is disposed in chamber 14 for the purpose of promoting flow of liquid into the chamber, as will be explained in more detail hereinbelow. Cup 12 includes structure at its upper end defining a liquid inlet opening 17 that is in fluid communication with the interior of chamber 14.

Device 10 also includes porous capture media means in the form of a circular element 18 for capturing and displaying products of an immunoassay procedure, a function which will be described in greater detailed hereinafter. Element 18 is disposed adjacent inlet opening 17 and operationally is in fluid communication with absorbent plug 16 during the conduct of the immunoassay procedure. A circular, hydrophobic, porous separator element 20 may be interposed between element 18 and absorbent plug 16 to facilitate essentially complete separation of said reaction products and liquid residues. Again, the function and characteristics of separator 20 will be described in greater detail hereinbelow.

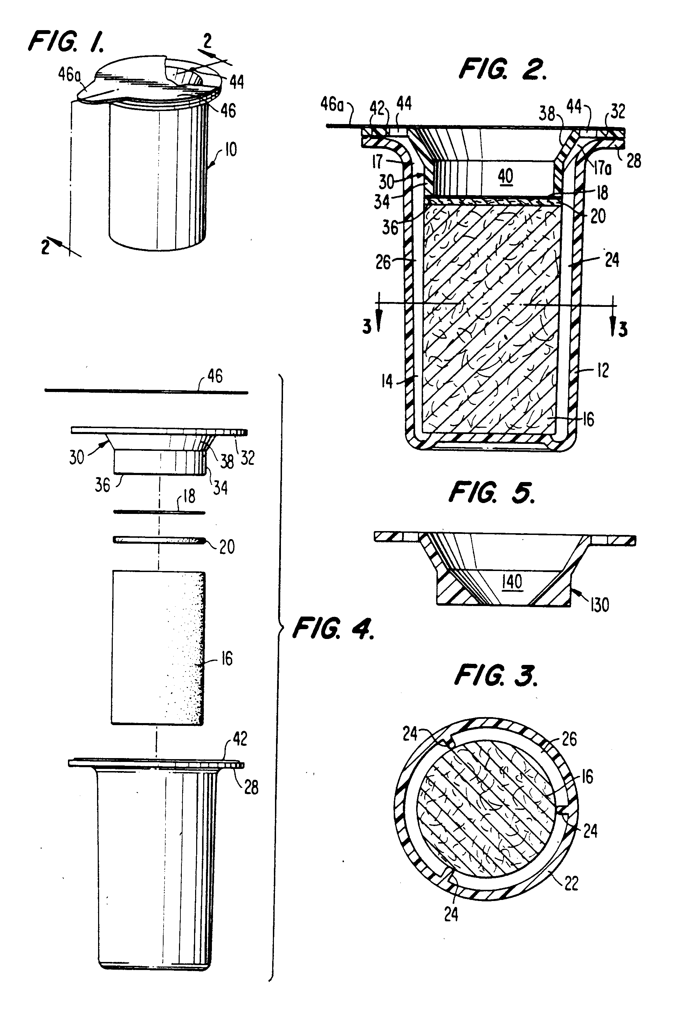

Cup 12 comprises a cylindrical wall 22 which surrounds chamber 14. A plurality of elongated, generally rectangular wing members 24 extend radially inwardly from wall 22, as can best be seen in Fig. 3. The innermost edge of each member 24 contacts the outer periphery of absorbent plug 16, and thus, wing members 24 present spacer means disposed in chamber 14 for holding absorbent plug 16 away from the inner surfaces of wall 22 to provide a breathing space 26 which communicates with inlet 17 and extends essentially entirely around the circumferential periphery of absorbent plug 16.

Cup 12 is also provided with an annular flange 28 which extends around inlet 17. Device 10 further includes a generally annular lid element 30 having a mating, corresponding flange 32. Element 30 also comprises a cylindrical member 34 having an annular surface 36 at its free end. A hollow, generally frustoconical segment 38 interconnects cylindrical member 34 and flange 32. Together, cylindrical member 34 and frusto-conical segment 38 present means defining a throat 40 which extends through inlet 17. Functionally, throat 40 is positioned for directing the flow of liquid reaction medium onto the porous capture media element 18.

Flange 28 extends around inlet 17 and flange 32 extends around throat 40. Flanges 28 and 32 are mating, corresponding flanges, and when the device is assembled as illustrated in Fig. 2, annular surface 36 is in tight, pushing contact with respect to porous capture media element 18. In this connection, it is to be understood that absorbent plug 16 is preferably somewhat resilient and compressible, and during assembly surface 36 contacts element 18 and pushes the latter toward absorbent plug 16 and into tight fluid communication therewith by way of the pores of separator element 20.

During assembly, flanges 28 and 32 are held together in mated relationship with throat 40 aligned with inlet 17 and with annular surface 36 held tightly against element 18 in pushing relationship thereto. Accordingly, cylindrical member 34 and its free annular surface 36 present a pusher element disposed in pushing relationship relative to the porous media capture element 18. Manifestly, during assembly, the flanges 28 and 32 are held together by a mechanical device (not illustrated) and while so held together, the same are preferably sonically welded. To facilitate such procedure, means in the nature of annular sonic weld energy director beads 42 are initially provided on flange 28 to facilitate the sonic welding process. However, it is to be understood that sonic welding and beads 42 are not critical aspects of the present invention. And in fact, there are alternative methods for securing flanges 28 and 32 together, such alternative methods being conventionally used and known to those skilled in the art.

A pair of spaced, opposed vent holes 44 are provided in flange 32, as can best be seen in Fig. 2. Vent holes 44 are disposed in fluid communication with breathing space 26 via the annular portion 17a of inlet 17 that surrounds cylindrical member 34 and frusto-conical segment 38 of lid element 30. Vent holes 44 are thus available to facilitate venting of breathing space 26 to the atmosphere surrounding the device 10 during the conduct of an immunoassay procedure utilizing device 10.

As can best be seen viewing Fig. 2, the external openings of vent holes 44 and the external opening of throat 40 are all disposed in a single plane extending along the upper surface of flange 32. Sealing means in the nature of a single planar foil element 46 is removably affixed to the upper surface of flange 32 using conventional adhesive means. Foil element 46 is provided with a conventional tab 46a to facilitate removal of element 46 from the upper surface of flange 32 by peeling. Accordingly, foil element 46 closes throat 40 and vent holes 44 to thereby prevent introduction of foreign material into the device prior to its intended use and yet foil element 46 is readily removable by the user at the time the device 10 is to be used.

Cup 12 and lid element 30 may be constructed of plastic or glass or any other suitable material, and these elements may preferably be made by an injection molding procedure utilizing a thermoplastic material. The only limitation on the materials of construction being that the same must be inert to the reactants and reaction products.

Absorbent plug 16 is an absorbent member having capillary passages extending therethrough in a diversity of directions which are both transverse to and generally parallel to the surface at the upper and lower ends of plug 16. There are a number of materials which are well known to those of ordinary skill in the art to which this invention pertains that may be used to construct absorbent plug 16. Such materials include hydrophillic polymers, particulate absorbents, glass fibers, cotton fibers, cellulose fibers, wood pulp and/or sponge. Other materials which may find use as plug 16 include polysaccharides, for example cellulosic materials, such as paper and cellulose acetate. Cellulose acetate fibers arranged in the same manner as in a cigarette filter may be utilized to construct absorbent plug 16. Another useful material is the absorbent material used in a tampon. A particularly useful material is a cellulose acetate fiber Transorb(TM) plug as manufactured by American Filtrona Co. In any event, the important features of the materials useful in the construction of plug 16 are simply that the same be capable of absorbing aqueous materials and that they possess sufficient structural integrity to permit the initial construction of the device. Further useful absorbent materials are disclosed, for example, in United States letters patent nos. 4,246,339; 4,623,461; 4,632,901; and 4,366,241.

Separator element 20 simply serves the purpose of separating porous capture media element 18 from absorbent plug 16 to assure that liquid at the upper surface of absorbent plug 16 does not interfere with the results to be evaluated at the surface of element 18. In this respect, separator element 20 should preferably be constructed of a hydrophobic, porous material, such as, for example, the hydrophobic, porous non-woven rayon material disclosed in U.S. 4,246,339 or the porous polyethylene or other material which does not bind receptor non-specifically, such as is disclosed in International Publication No. WO85/05451 (International Application No. PCT/US85/00870). A porous polyester material available commercially in sheet form from Porex Technologies is a particularly useful material. Other useful materials include glass fiber layers (Whatman(TM)) and porous plastic layers (Porex(TM) or Pellon(TM)).

With regard to the porous capture media element 18, this element may take any one of several different forms depending on the type of immunoassay procedure which is utilized. For example, if the immunoassay involves an ELISA technique, element 18 may be a membrane having a co-reactant for the reagent to be assayed in the test liquid sample immobilized on the internal and external surfaces thereof. Such membranes are utilized in the procedures disclosed in United States letters patent no. 4,246,339 and in said International Publication no. WO85/05451. The membranes useful in connection with such procedure are fully disclosed and described in United States letters patent no. 4,340,479. Manifestly, methods for binding immunoreactants to such membranes are well known to those skilled in the art to which the present invention pertains.

In another form of the invention, the porous capture media element 18 may be composed of such things as glass fiber filters (Whatman(TM) GF/A), regenerated cellulosic membranes (Schleicher and Schuell) and microporous membranes (Millipore MF series membranes HAWP, SSWP, SMWP and SCWP with pore sizes of 0.45, 3, 5 and 8 µm respectively). All of these materials have been successfully utilized for capturing and collecting a solid phase product resulting from an immunoassay procedure. Manifestly, in such process the porous capture media element simply comprises a filter element having pores of a size to prevent passage of desired reaction products. Accordingly, the desired reaction products accummulate on the surface of the element and are available there for visual inspection.

Another capture material which is potentially useful as element 18 is a microporous member to which avidin has been linked whereby biotinylated reaction materials may be captured.

In the operation of device 10, immunoreagents are contacted with a sample and the mixture is allowed to incubate for a period of time, all as is well known to those skilled in the immunoassay art. After a sufficient incubation period, the reaction mixture is simply poured through throat 40 and onto element 18. Capillary action in absorbent plug 16 promotes flow of liquid through element 18 and through separator 20, and thus the liquid phase is separated from the solid phase by element 18. Air or other gases originally present in plug 16 are readily vented through space 26 and upwardly through vent holes 44. This facilitates the absorbent action in absorbent plug 16, since with the structure provided by the present invention, there is no substantial possibility of entrapping air within the device to impede the flow of fluid into absorbent plug 16. To insure complete separation between the liquid phase in absorbent plug 16 and the solid phase on the surface of porous media capture element 18, a rinse solution may be poured through throat 40 after the reaction mixture has been introduced. Manifestly, formation of coloration on the surface of element 18 provides an almost instantaneous indication of the results of the assay, whether the same involves a metal sol tag in accordance with the procedure disclosed in said co-pending Cole et al. application, or an enzyme tag and an immobilized antibody, such as is disclosed in United States Letters Patent No. 4,407,943.

It will be readily apparent to those of ordinary skill in the art, that while the device of the present invention has been described in connection with certain specific immunoassay procedures, the same may also be utilized in connection with other immunoassay procedures that involve a liquid phase reaction and production of a labelled immunoreaction product that is capturable on a porous media capture element, either by a filtration process or chemical linkage. In this regard, element 18 may be a filter element having pores of a size to prevent passage of a desired reaction products, or a porous element, such as a microporous membrane, to which is bound an immunoreactive substance that is specifically reactive in the desired immunoassay reaction.

Sealing element 46 may simply be a formed piece of an impermeable sheet material. Preferably element 46 may be a piece of metal foil which is removably attached by an adhesive to the upper surface of flange 32 in closing relationship to throat 40 and vent holes 44. In this regard, the attachment may preferably be by way of an adhesive material which permits foil 46 to simply be peeled away from the top of device 10 utilizing tab 46a to thereby to expose throat 40 and vent holes 44. While foil 46 remains in place, all internal areas of device 10 are protected against inadvertent introduction of foreign materials. Accordingly, the transportability, shelf life and storability of the device is enhanced.

With reference to figure 5, an alternative lid element 130 is illustrated. Lid element 130 is generally the same as lid element 30 except that it is designed so that throat area 140 is frusto-conical in shape. Thus, the liquid flowing through throat 140 is channeled into a much smaller flow area and therefore contacts a much smaller area of porous capture medium 18. This results in a concentration of the tagged reaction product captured on medium 18 and a resultant enhancement of coloration. Lid element 130 may be used to replace lid element 30, particularly in connection with the form of the invention where the porous capture medium 18 is a simple filter and the tag is a metal sol particle as disclosed in said co-pending Cole et al. application.

## Claims

1. A disposable, pre-packaged device for conducting an immunoassay procedure which results in the production of separable liquid and solid phases, said device comprising:
a cup means (12) defining an elongated chamber (14) for receiving the liquid phase from said procedure, said cup means (12) comprising a cylindrical wall extending around said chamber (14), a closure wall at one end of the chamber and a liquid inlet (40) communicating with the chamber (14);
a generally cylindrically shaped absorbent plug (16) disposed entirely within the chamber (14) for absorbing said liquid phase, said plug (16) having a lower surface resting on said closure wall, a circumferential peripheral surface extending therearound and an upper flat surface disposed in alignment with said inlet;
a porous capture media means (18) for capturing and displaying a solid phase separated from said liquid phase, said porous capture media means (18) being disposed between said inlet (40) and said flat surface and being in fluid communication with said absorbent plug (16) via said flat surface, whereby said absorbent plug (16) promotes flow of liquid phase through said capture media means (18) during conduct of an immunoassay procedure,
**characterized** in that a spacer means (24) is provided in the chamber (14) holding the peripheral surface of the absorbent plug (16) away from the cylindrical wall to thereby present an annular breathing space (26) communicating directly with the peripheral surface of the absorbent plug (16) and extending essentially entirely around said peripheral surface.

2. A device as set forth in claim 1, characterized by a lid element (30) mounted on the cup means (12) adjacent the inlet (40) and having a centrally disposed, elongated cylindrical member (34) extending through the inlet (40), said element (30) having an opening extending through the cylindrical member (34) for directing flow of liquid assay reaction medium through the inlet (40) and onto the capture means (18) and including at least one vent hole (44) adjacent said inlet (40) in communication with said space (26) to facilitate venting of the absorbent plug (16) via said peripheral surface and said space (26) as the plug (16) absorbs a liquid phase.

3. A device as set forth in claim 2, characterized in that said cylindrical member (34) includes a pusher segment (36) for contacting said capture means (18) and exerting a pushing force against the latter to hold the capture means (18) and the plug (16) in said fluid communication.

4. A device as set forth in claim 2 or 3, characterized in that said lid element (30) includes an annular flange (32) extending around said opening and said cup means (12) includes a mating, corresponding annular flange (28) extending around said inlet (40) said device including means for securing the flanges together in mated relationship with the opening aligned with the inlet (40).

5. A device as set forth in one of the preceding claims, characterized in that each vent hole (44) is disposed in said lid element flange (32).

6. A device as set forth in one of the preceding claims, characterized in that it comprises romovable sealing means (46) closing the opening (40) and each vent hole (44) to thereby prevent introduction of foreign material into the device prior to intended use.

7. A device as set forth in claim 6, characterized in that the external portions of the opening (40) and the vent hole(s) (44) are disposed in a common plane and said sealing means (46) comprising a single planar element disposed in sealing relationship to said external portions.

8. A device as set forth in one of the claims 3 to 7, characterized in that said pusher segment (36) is cylindrical and has an annular surface at its free end disposed for contacting the porous capture media means (18), said opening (40) extending through the cylindrical pusher segment (36).

9. A device as set forth in one of the preceding claims, characterized in that said porous media means (18) comprises a filter element having pores of a size to prevent passage of desired reaction products.

10. A device as set forth in one of the claims 1 to 8, characterized in that said porous media means (18) comprises a microporous membrane to which is bound an immunoreactive substance that is specifically reactive in the desired reaction.

11. A device as set forth in one of the preceding claims, characterized in that a porous separator element (20) is interposed between the porous means (18) and the flat surface of the absorbent plug (16).

12. A device as set forth in claim 11, wherein said separator element (20) is constructed of a hydrophobic material.

13. A device according to one of the preceding claims, characterized in that said plug (16) is generally circular in cross-sectional configuration and said spacer means (24) comprises a plurality of general rectangular shaped wing members extending radially inwardly from said cylindrical wall.

## Patentansprüche

1. Vorverpackte Einwegvorrichtung für Immuntest-Prozeduren, was zur Erstellung von trennbaren Flüssig- und Feststoffphasen führt, wobei die besagte Vorrichtung einen Becher (12) umfaßt, der einen langgezogenen Raum bzw. eine längliche Kammer (14) für die Aufnahme der Flüssigphase aus der besagten Prozedur aufweist, und der besagte Becher (12) eine zylindrische Wandung aufweist, die sich um die besagte Kammer (14) herumerstreckt, eine Abschlußwandung an einem Ende der Kammer und ein Flüssigkeitseinlaß (40), der mit der Kammer (14) in Verbindung steht und die Vorrichtung weiterhin einen im allgemeinen zylindrisch geformten Saugstopfen (16) aufweist, der die Kammer (14) voll ausfüllt, um die besagte Flüssigkeitsphase aufzusaugen und der besagte Stopfen (16) eine untere Fläche hat, die auf der besagten Verschlußwandung ruht, eine sich darum erstreckende, periphere Umfangsfläche und eine obere, ebene Fläche, die mit dem besagten Einlaß bündig ist und die Vorrichtung weiterhin eine poröse Auffangeinrichtung (18) für das Auffangen und die Anzeige einer Feststoffphase aufweist, die von der Flüssigphase getrennt ist, und sich die besagte poröse Auffangeinrichtung (18) zwischen dem besagten Einlaß (40) und der besagten ebenen Fläche befindet und über die besagte ebene Fläche mit dem besagten Saugstopfen (16) flüssig in Verbindung steht, so daß der besagte Saugstopfen (16) den Fluß der Flüssigphase durch die besagte Auffangeinrichtung (18) während der Durchführung einer Immuntestprozedur fördert, was dadurch gekennzeichnet ist, daß sich ein Abstandsstück (24) in der Kammer (14) befindet, das die periphere Fläche des Saugstopfens (16) von der zylindrischen Wandung entfernt hält, um somit einen ringförmigen Atmungs- oder Lüftungsraum (26) zu bilden, der direkt mit der peripheren Fläche des Saugstopfens (16) in Verbindung steht und sich im wesentlichen gänzlich um die besagte periphere Fläche herum erstreckt.

2. Vorrichtung gemäß Anspruch 1, dadurch gekennzeichnet, daß sich ein Deckel (30) auf dem Becher (12) im Anschluß an den Einlaß (40) befindet und einen mittig angeordneten, länglichen, zylindrischen Teil (34) aufweist, der sich durch den Einlaß (40) erstreckt, wobei der besagte Deckel (30) eine Öffnung aufweist, die sich durch den zylindrischen Teil (34) erstreckt, um den Fluß des flüssigen Testreaktionsmittels durch den Einlaß (40) und zur Auffangeinrichtung (18) leitet und zumindest eine Lüftungsöffnung (44) im Anschluß an den besagten Einlaß (40) aufweist, die mit dem besagten Raum (26) in Verbindung steht, um ein Lüften des Saugstopfens (16) über die besagte periphere Fläche und den besagten Raum (26) zu erleichtern, während der Stopfen (16) eine Flüssigphase aufsaugt.

3. Vorrichtung gemäß Anspruch 2, dadurch gekennzeichnet, daß der besagte zylindrische Teil (34) ein Drückersegment (36) für den Kontakt mit der besagten Auffangeinrichtung (18) aufweist und eine Druckkraft gegen letztere ausübt, um die Auffangeinrichtung (18) und den Stopfen (16) in der besagten flüssigen Verbindung zu halten.

4. Vorrichtung gemäß Anspruch 2 oder 3, dadurch gekennzeichnet, daß der Deckel (30) einen ringförmigen Flansch (32) umfaßt, der sich um die besagte Öffnung herum erstreckt, und der besagte Becher (12) einen entsprechenden, dazu passenden ringförmigen Flansch (28) umfaßt, der sich um den besagten Einlaß (40) herumerstreckt, wobei die besagte Vorrichtung Mittel oder Möglichkeiten für die Befestigung der beiden zusammenpassenden Flansche mit der mit dem Einlaß (40) bündig ausgerichteten Öffnung umfaßt.

5. Vorrichtung gemäß einem der obigen Ansprüche, dadurch gekennzeichnet, daß sich die Lüftungsöffnung (44) in besagtem Deckelflansch (32) befindet.

6. Vorrichtung gemäß einem der obigen Ansprüche, dadurch gekennzeichnet, daß diese entfernbare Dichtungsmittel (46) aufweist, die die Öffnung (40) und jede Lüftungsbohrung (44) verschließen, um somit das Eindringen von Fremdstoffen in die Vorrichtung vor dem beabsichtigten Einsatz verhindern.

7. Vorrichtung gemäß Anspruch 6, dadurch gekennzeichnet, daß die außenliegenden Teile der Öffnung (40) und der Lüftungsbohrung(en) (44) in einer gemeinsamen Ebene liegen und die besagten Dichtungsmittel (46) eine einzelne Fläche umfassen, die die besagten außenliegenden Teile abdichten.

8. Vorrichtung gemäß einem der Ansprüche 3 bis 7, dadurch gekennzeichnet, daß das Drückersegment (36) zylindrisch ausgeführt ist und an seinem freien Ende über eine ringförmige Fläche für den Kontakt mit der porösen Auffangeinrichtung (18) verfügt, wobei sich die besagte Öffnung (40) durch das zylindrische Drückersegment (36) erstreckt.

9. Vorrichtung gemäß einem der obigen Ansprüche, dadurch gekennzeichnet, daß die besagte poröse Auffangmöglichkeit (18) einen Filter umfaßt, der Poren einer solchen Größe aufweist, daß ein Durchlaß der gewünschten Reaktionsprodukte verhindert wird.

10. Vorrichtung gemäß einem der Ansprüche 1 bis 8, dadurch gekennzeichnet, daß die poröse Auffangeinrichtung (18) eine mikroporöse Membran aufweist, woran eine immunreaktive Substanz gebunden ist, die sich bei der gewünschten Reaktion als besonders reaktiv erweist.

11. Vorrichtung gemäß einem der obigen Ansprüche, dadurch gekennzeichnet, daß ein poröses Trennelement (20) zwischen der porösen Auffangeinrichtung (18) und der ebenen Fläche des Saugstopfens (16) angebracht ist.

12. Vorrichtung gemäß Anspruch 11, worin das besagte Trennelement (20) aus hydrophobem Material erstellt ist.

13. Vorrichtung gemäß einem der obigen Ansprüche, dadurch gekennzeichnet, daß der besagte Stopfen (16) im allgemeinen querschnittsmäßig kreisförmig ausgebildet ist und daß das besagte Abstandsstück (24) eine Vielfalt von allgemein rechtwinklig geformten Flügeln umfaßt, die sich von der besagten zylindrischen Wandung radial nach innen erstrecken.

## Revendications

1. Dispositif jetable préemballé pour exécuter un procédé d'essai immunologique résultant en la production de phases liquide et solide séparées, le dit dispositif comprenant:
un récipient (12) qui définit une chambre allongée (14) et qui reçoit la phase liquide du dit procédé, le dit récipient (12) comprenant une paroi cylindrique s'étendant autour de la dite chambre (14), une paroi de fermeture à une extrémité de la chambre et une admission pour le liquide (40) qui communique avec la chambre (14);
un tampon absorbant de forme généralement cylindrique (16) disposé entièrement à l'intérieur de la chambre (14) permettant l'absorption de la dite phase liquide, le dit tampon (16) possèdant une surface inférieure reposant sur la dite paroi de fermeture, une surface périphérique circonférentielle s'etendant tout autour de la chambre et une surface supérieure plane alignée par rapport à la dite admission;
un agent de capture poreux (18) permettant la capture et l'étalement de la dite phase solide séparée de la dite phase liquide, le dit agent de capture poreux (18) étant disposé entre la dite admission (40) et la dite surface plane et étant en communication fluidique avec le dit tampon absorbant (16) via la dite surface plane, de sorte que le dit tampon absorbant stimule le passage de la phase liquide à travers le dit agent de capture (18) pendant l'exécution d un procédé d'essai immunologique.
**caractérisé en ce que** un agent espaceur (24) est prévu dans la chambre (14) afin de maintenir la surface périphérique du tampon absorbant (16) séparée de la paroi cylindrique, de sorte à présenter ainsi un espace de respiration (26) communiquant directement avec la surface périphérique du tampon absorbant (16) et s'étendant essentiellement entièrement autour de la dite surface périphérique.

2. Un dispositif selon la revendication 1, caractérisé par un élément couvercle (30) monté sur le récipient (12) adjacent à l'admission (40) et possédant une partie cylindrique disposée de manière centrale (34) passant à travers l'admission (40), le dit élément (30) présentant une ouverture s'étendant à travers la partie cylindrique (34) permettant de diriger l'agent de réaction liquide de l'essai à travers l'admission (40) et sur l'agent de capture (18) et comportant au moins un orifice d'aération (44) adjacent à la dite admission (40) en communication avec le dit espace (26) afin de faciliter l'aération du dit tampon absorbant (16) via la dite surface périphérique et le dit espace (26) lorsque le tampon (16) absorbe une phase liquide.

3. Un dispositif selon la revendication 2, caractérisé en ce que la dite pièce cylindrique (34) comporte un segment poussoir (36) permettant d'entrer en contact avec le dit agent de capture (18) et d'exercer une force de pression contre ce dernier afin de maintenir la dite communication fluidique entre l'agent de capture (18) et le tampon (16).

4. Un dispositif selon la revendication 2 ou 3, caractérisé en ce que le dit élément couvercle (30) comporte une bride annulaire (32) s'étendant autour de la dite ouverture et que le dit récipient (12) comporte une bride annulaire correspondante conjuguée (28) s'étendant autour de la dite admission (40), le dit dispositif comportant des systèmes permettant de fixer ensemble les brides de manière conjuguée de sorte que l'ouverture soit alignée avec l'admission (40).

5. Un dispositif selon l'une des revendications ci-dessus, caractérisé en ce que le dit orifice de ventilation (44) est disposé dans la dite bride de l'élément couvercle (32).

6. Un dispositif selon l'une des revendications ci-dessus, caractérisé en ce qu'il comporte des agents d'étanchéité amovibles (46) fermant l'ouverture (40) et chaque orifice de ventilation (44) afin d'empêcher l'introduction de matières étrangères dans le dispositif avant son utilisation intentionnelle.

7. Un dispositif selon la revendication 6, caractérisé en ce que les parties externes de l'ouverture (40) et l'orifice (ou les orifices) de ventilation (44) sont disposés sur un même plan, et que les dits agents d'étanchéité (46) comportent un élément situé dans un seul plan possédant une relation d'étanchéité avec les dites parties externes.

8. Un dispositif selon l'une des revendications 3 à 7, caractérisé en ce que le dit élément poussoir (36) est cylindrique et présente une surface annulaire à son extrémité libre située de sorte à entrer en contact avec l'agent de capture poreux (18), la dite ouverture (40) s'étendant à travers le segment poussoir cylindrique (36).

9. Un dispositif selon l'une des revendications ci-dessus, caractérisé en ce que le dit agent poreux (18) comporte un élément de filtrage présentant des pores dont la taille permet d'éviter le passage des produits de réaction désirés.

10. Un dispositif selon l'une des revendications 1 à 8, caractérisé en ce que le dit agent poreux (18) comporte une membrane microporeuse à laquelle est liée une substance immuno-réactive réagissant spécifiquement dans la réaction désirée.

11. Un dispositif selon l'une des revendications ci-dessus, caractérisé en ce qu'un élément séparateur poreux (20) est interposé entre l'agent poreux (18) et la surface plane du tampon absorbant (16).

12. Un dispositif selon la revendication 11, où le dit élément séparateur (20) est fabriqué à partir d'un matériau hydrophobe.

13. Un dispositif selon l'une des revendications ci-dessus, caractérisé en ce que le dit tampon (16) est généralement circulaire dans sa configuration transversale, et que le dit agent espaceur (24) comporte une série de parties latérales de forme généralement rectangulaire s'étendant de manière radiale vers l'intérieur à partir de la dite paroi cylindrique.
